# EUROPÄISCHE PATENTANMELDUNG

(11) **EP 0 878 131 A2**
(43) Veröffentlichungstag der Anmeldung: **18.11.1998**
(21) Anmeldenummer: 98104880.4
(22) Anmeldetag: 18.03.1998
(51) Int. Cl.: A21D 2/02, A61K 7/00, A23K 1/00, A01K 1/00

(54) **Gesundheitsprodukt für den täglichen Bedarf**

(30) Priorität: 18.03.1997 DE 19711073
(71) Anmelder: Widdascheck, Christian, 20255 Hamburg (DE)
(72) Erfinder: Widdascheck, Christian, 20255 Hamburg (DE)

(57) **Zusammenfassung**

Die in der Lebensmittelbranche, bei der Filtration von flüssigen Lebensmitteln, besonders in Brauereien bei der Bierfiltration, verwendete Kieselgur wird bislang nach der Filtration in Kläranlagen oder über Deponien als Abfallstoff entsorgt. Allenfalls wird sie in seltenen Fällen auf lanwirtschaftlichen Flächen als Düngemittel verwendet. Eine Regeneration zur erneuten Filtration in der Lebensmittelbranche ist praktikabel aber problematisch. Demgegenüber wird eine Verwertung dieses Abfallstoffes als Bestandteil von Gesundheitsprodukten für den täglichen Bedarf vorgeschlagen. Dabei können die organischen Bestandteile, vor allem die Hefe, der mineralische Bestandteil, oder beide zusammen vorteilhaft eingesetzt werden. Beispiele für Produkte, die nach der Erfindung gebrauchte Filtrationskieselgur enthalten, sind Tierpflegemittel, insbesondere ein Katzenstreu und ein Beifuttermittel; Körperpflegemittel, wie eine Handwaschpaste oder Peelingcremes; und die Verwendung von gebrauchter Filtrationskieselgur als Backzusatz in Brot.

## Beschreibung

Die Erfindung betrifft die Verwendung von Kieselgur, die allgemein für die Filtration von flüssigen Lebensmitteln, insbesondere in Brauereien als Filterhilfsmittel für die Bierfiltration verwendet wird. Kieselgur ist eine in natürlichen Lagerstätten vorkommende Ablagerung von kleinen Schalentieren ehemaliger Meere oder Seen, sogenannten Kieselalgen (Diatomeen), aus dem Tertiär und Quartär. Die den Betrieben zur Filtration angelieferte Kieselgur ist bereits ein qualitativ hochwertiges Produkt. Sie wird aus der Rohgur durch erhitzen, teilweise bis zu 1.200 Grad Celsius, d. h. sehr energieaufwendig, sowie durch Vermahlung, Selektionierung und Reinigung gewonnen. Beim Filtriervorgang, beispielsweise in der Brauerei, lagern sich Hefe und andere ungelöste organische Bestandteile an den sehr aufnahmefähigen Kieselgurpartikeln an, wodurch aus dem ursprünglich weiß bis hellbraunen Pulver eine dunkelbraune pastöse Masse entsteht, die ein Abfallprodukt ist und entsorgt werden muß.

Im Jahr 1995 fielen in Deutschland allein bei der Bierherstellung 72000 Tonnen gebrauchte Kieselgur an, die zum größten Teil durch Kläranlagen oder Deponien entsorgt wurde. In den anderen Bereichen der Lebensmittelbranche, in denen Kieselgur als Filtermaterial verwendet wird, fallen nochmals 25000 bis 45000 Tonnen an, die zumeist auf gleiche Weise entsorgt werden. Dies ist ökologisch und ökonomisch nicht sinnvoll.

Bekannt ist lediglich eine Verwertung als Dünger, wobei hauptsächlich der Stickstoffanteil in der angelagerten organischen Substanz ausgenutzt wird. Hierüber veröffentlichten R. Schildbach und W. Ritter 〉〉Brauerei-Kieselgur〈〈, Berichte aus der Wissenschaftsförderung der deutschen Brauwirtschaft e. V. Berlin, Mai 1995. Die Regeneration zur erneuten Filtration in der Lebensmittelbranche ist praktikabel aber problematisch.

Der Erfindung liegt die Aufgabe zugrunde, für die als Filterhilfsmittel bei der Filtration von flüssigen Lebensmitteln, insbesondere Bier, anfallende, mit Hefe und anderen ungelösten organischen Partikeln beladene Kieselgur, eine sinnvolle umweltschonende Verwendung zu finden.

Diese Aufgabe wird erfindungsgemäß durch den Patentanspruch 1 angegebene Anwendung gelöst. Dabei werden teils die Eigenschaften der Kieselgur, teils die Eigenschaften der an ihr angelagerten Hefe oder anderer organischen Substanzen, oder beide, gemeinsam genutzt. Vor allem aber bietet die Hygiene und große Reinheit dieses Abfallprodukts, dessen chemische Zusammensetzung laufend geprüft und dokumentiert wird, die Möglichkeit einer Verwendung im Zusammenhang mit Nahrungs- und Futtermitteln oder unmittelbar am menschlichen oder tierischen Körper. Entsprechende Produkte können deshalb zusammenfassend als Gesundheitsprodukte bezeichnet werden. Die große Verbreitung des Filtermaterials Kieselgur läßt kurze Wege und eine schnelle Verwendung der anfallenden Kieselgur in den Weiterverarbeitungsstätten erwarten.

Beispiele bevorzugter Produkte, die nach der Lehre der Erfindung gebrauchte Kieselgur, z. B. aus Brauereien enthalten, werden nachstehend beschrieben.

### 1. Hefebrot

Ein angenehm schmeckendes und ernährungsphysiologisch hochwertiges Brot kann aus folgenden Zutaten hergestellt werden:
- 300 g: Mehl
- 100 g: Hirse
- 100 g: feine Kieselgur (beispielsweise bei 35 °C getrocknet)
- 8 g: Trockenhefe
- 500 g: Flüssigkeit

Die frische gebrauchte Brauerei-Kieselgur wird entweder ungetrocknet verwendet, oder bei 35 Grad Celsius langsam getrocknet, um die Denaturierung der Hefe zu verhindern. Zu diesem Zweck ist auch eine Vakuumtrocknung oder Gefriertrocknung möglich. Die Kieselgur sollte anschließend fein gemahlen oder gemörsert werden. Die Brotzubereitung und das Backen erfolgt im übrigen in bekannter Weise.

### 2. Bierhefe-Kieselgur-Tabletten

Der gesamte Herstellungsprozeß sollte in einem Milieu ablaufen, in dem die Organik nicht denaturiert, um die gesundheitsfördernde Wirkung dieser zu erhalten. Für 700 Tabletten wird ein Feuchtgranulat bereitet aus
- 100 g: Kieselgur (35°C getrocknet)
- 100 g: Gelatine-Lösung (10%ig)
- 30 g: Kartoffelstärke (Füllstoff).

Nach Trocknung bei 35°C werden 2,5 g Talkum als Formtrennmittel gleichmäßig über das Granulat verteilt. Das bestäubte Granulat wird in einer Tablettiermaschine verpreßt.

### 3. Handwaschpaste

In diesem Fall wird der Scheuereffekt der Kieselgur ausgenutzt. Die Kieselgur muß in einem Temperaturbereich getrocknet werden, der so hoch ist, daß die anhaftende Hefe degeneriert wird, da das Produkt sonst nicht haltbar ist. Das gleiche gilt für die Produktbeispiele 4 bis 8, deswegen wird in ihnen immer eine Kieselgur verwand, die bei mindestens 105°C getrocknet wurde, da dadurch eine Denaturierung der an ihr angelagerten Hefe garantiert ist. Aus diesen Gründen kann es sinnvoll sein in den Beispielen 3 bis 8 ausgebrannte Kieselgur zu verwenden, da so eventuelle negative Einflüsse der Organik ausgeschlossen werden können.

Man gibt 35 ml destilliertes Wasser in ein Becherglas und erwärmt auf 50 bis 60°C. Dann wird etwa 1 g des Verdickungsmittels Rewoderm zugeführt und umgerührt, bis es sich gleichmäßig verteilt hat. Nun werden Tenside, z. B. 12 ml Zeterol und 5 ml Tegobetain L7 dazugegeben, danach je 5 Tropfen Nutrilan und Heliozimt sowie einige Tropfen Parfümöl. Schließlich werden je nach gewünschtem Reibeeffekt 10 bis 50 g Kieselgur oder mehr zugegeben.

### 4. Peeling-Creme

Eine solche Creme reinigt und pflegt gleichzeitig. Mit ihrer Hilfe werden Talgabsonderungen und abgestorbene Hautzellen auf sanfte Art entfernt. Dabei sind die Kieselgurpartikel ebenfalls nützlich. Außerdem ist eine besondere Hautverträglichkeit der Brauerei-Kieselgur dadurch gegeben, daß diese nach der Trocknung bei 105°C durchschnittlich 0,72% Fett enthält und einen pH-Wert von 5,7 aufweist.

Typische Bestandteile solcher Cremes sind Vaselinöl, Bienenwachs, wasserfreies Lanolin, Kamillenextrakt, Cethylalkohol u. a. Das üblicherweise ebenfalls enthaltene Mandelschalengranulat wird nach der Erfindung durch bei mindestens 105°C getrocknete Brauerei-Kieselgur ersetzt und zwar mit einem Anteil von 10 bis 15 Gewichtsprozent oder mehr.

Eine andere, für ein erfrischendes Peeling-Gel zur Fußmassage geeignete Rezeptur lautet:
- 1 Teil: Polyacrylsäure
- 12 Teile: Propylenglycol
- 16 Teile: Natriumhydroxydlösung (1%ig)
- 17 Teile: Aqua dest.

Hier werden in einem Ansatz von 50 ml bis zu 50 g oder mehr der bei mindestens 105°C getrockneten Brauerei-Kieselgur eingerührt.

### 5. Gesichtspuder

Puder enthält üblicherweise Talkum. Dieser kann durch Brauerei-Kieselgur ersetzt werden, in Anbetracht der schon genannten Hautverträglichkeit dieses hochwertigen Abfallprodukts.

Ein bevorzugtes Rezept lautet:
- 8 g: Kieselgur (mindestens 105°C getrocknet)
- 6 g: Seidenweiß (Titandioxyd)
- 4 g: Magnesiumstearat
- 1-2 g: Pigmentmischung
- 1-3 g: Jojobaöl
- 4 g: Parfümöl

Die Pigmentmischung, das Jojobaöl und das Parfümöl werden miteinander zu einer Paste vermengt. Kieselgur, Seidenweiß und Magnesiumstearat werden ebenfalls vermischt. Dann wird löffelweise die Pudermischung in die Farbpaste gegeben, bis eine lockere Pudermischung entsteht, in der Parfümöl und Jojobaöl gleichmäßig verteilt sind.

Die Puderbasis kann auch Kartoffelstärke enthalten. Zum Beispiel mit Perlglanzpigment und entsprechenden Farbpigmenten kann sie als Lidschattenpuder weitergebildet werden.

### 6. Trockenshampoo

Dieses Produkt besteht üblicherweise zum Teil aus Talkum und ggfs. Weizenstärke. Diese sollen u. a. Feuchtigkeit und Talgabsonderungen aus den Haaren aufnehmen. Hier kommt das hohe Adsorptionsvermögen der Brauerei-Kieselgur zum Tragen, wenn damit bei einem an sich bekannten Rezept das Talkum und evtl. die Weizenstärke ersetzt wird:
- 10 g: Weizenstärke
- 5 g: Veilchenwurz
- 2,5 g: Brauerei-Kieselgur (mindestens 105°C getrocknet)
- 2,5 g: Bolus alba (Kolloidal-Kaolin)

### 7. Schnabelwetzstein für Ziervögel

Wenn sie mit Wasser vermischt werden, gehen Kieselgur und Kalk bei entsprechender Pressung eine feste Verbindung ein. So kann aus 5 Volumenteilen gebrauchte Brauerei-Kieselgur (getrocknet oder ausgebrannt) und einem Volumenteil Calciumhydroxydpulver mit Wasser eine Paste angerührt werden, aus der beim Verpressen der größte Teil des Wassers wieder austritt. Das enthaltene Calciumhydroxyd reagiert mit dem Kohlendioxyd der Luft nach einiger Zeit zu einer festen Verbindung.

Die enthaltene Kieselgur im Schnabelwetzstein wird bei dessen Verzehr teilweise über den Darm resorbiert und hat einen positiven Einfluß auf das Bindegewebe und das Gefieder der Tiere, die gesundheitsfördernde Wirkung der Bierhefe ist allgemein bekannt. Der nicht resorbierte Teil stellt einen wertvollen Ballaststoff dar. Entsprechend geformte Wetzsteine haben sich im Test durch Vogelliebhaber großer Beliebtheit erfreut. Den Ausgangsmaterialien können je nach Bedarf noch andere, dem Vogelorganismus dienliche Substanzen, beispielsweise weitere Vitamine oder jod, beigemischt werden

### 8. Tierstreu

Bei diesem Beispiel eines Hygieneprodukts, für das ein erheblicher Mengenbedarf besteht, wird die Adsorptionsfähigkeit für Flüssigkeiten und Gerüche der Kieselgur genutzt. Maßgebend hierfür ist die große äußere und innere Oberfläche der Kieselgur, sowie die CO2-Entwicklung der Hefe in den unteren Temperaturbereichen bei der Trocknung des Granulates bevor sie bei 105°C denaturiert. Ein bevorzugtes Tierstreu, z. B. Katzenstreu, besteht fast ausschließlich aus der Kieselgur, die noch frisch granuliert wird oder getrocknet mit einer 2%igen Methylzelluloselösung bzw. verdünnten Wasserglaslösung zu einem Teig vermengt und das Granulat dadurch gebunden wird. Die enthaltene Organik der gebrauchten Kieselgur ist für die Granulation und die Stabilität des Granulates ebenfalls entscheidend. Nach einem Granulieren wird das noch feuchte Streu getrocknet. Der Herstellungsprozess kann auch in einem Temperaturbereich ablaufen, bei dem der organische Anteil der Kieselgur verbrennt. Neben dem enormen Absorptionsvolumen zeichnet sich ein Tierstreu aus Kieselgur durch seine geringe Dichte aus, was vorteilhaft bei der Distribution und beim Transport des Endverbrauchers ist. Ein weiterer Vorteil ist die gute Entsorgbarkeit des gebrauchten Streus als Kompostzusatz, oder direkt als Dünger, aufgrund seiner fördernden Eigenschaften für Böden. Wo diese Entsorgungswege nicht möglich sind, kann das Streu auch portionsweise über die Toilette entsorgt werden. Dem Hauptrohstoff Kieselgur können problemlos Substanzen zur Desinfektion oder zur weiteren Absenkung des pH-Wertes der schon leicht sauren Filtrationskieselgur auf beispielsweise ca. 6,2 bis 5,8 zugefügt werden, um die Eigenschaften des Streus weiter zu steigern.

### 9. Beifuttermittel

Gebrauchte Filtrtationskieselgur kann als Beifuttermittel für Klein aber auch für Großtiere verwand werden. Die mineralische Kieselgur kann teilweise über den Darm resorbiert werden, vor allem durch die niedrigen pH-Wette in bestimmten Abschnitten des Verdauungstraktes. Die Kieselgur hat einen positiven Einfluß auf das Bindegewebe und das Fell der Tiere, die gesundheitsfördernde Wirkung der Bierhefe ist allgemein bekannt. Der nicht resorbierte Teil stellt einen wertvollen Ballaststoff dar. Optimal wäre die Verwendung der abfiltrierten Kieselgur als ganzes, durch eine entsprechend schonende Trocknung, bei der die gesundheitsfördernde Wirkung der Bierhefe erhalten bleibt.

Auch ein Ausbrennen der Kieselgur ist möglich und eine anschließende Zumischung von 10% Bierhefe oder mehr. Der Vorteil dieser Herstellungsweise liegt in einer weniger anspruchsvollen Behandlung der Kieselgur, da sie durch das Brennen wieder sterilisiert wird und eine längere Haltbarkeit des Endproduktes garantiert.

Dem Beifuttermittel können noch andere gesundheitsfördernde Stoffe beigemischt werden. Das Beifuttermittel kann in pudriger oder granulierter Form dem Futter untergemischt werden.

## Patentansprüche

1. Verwendung von als Filterhilfsmittel bei der Filtration von flüssigen Lebensmitteln, besonders die des Bieres, angefallener, mit Hefe und anderen ungelösten organischen Partikeln beladener Kieselgur, als Bestandteil von Gesundheitsprodukten für den täglichen Bedarf, nämlich von Nahrungsmitteln, von Futtermitteln, von Mitteln zur Körper- und Schönheitspflege und von Tierpflegemitteln.

2. Brot, gekennzeichnet durch einen Anteil von 1% bis 50% in TrS. Kieselgur aus der Filtration von flüssigen Lebensmitteln.

3. Brot, gekennzeichnet durch einen Anteil von 1% bis 50% in TrS. Kieselgur aus der Filtration von Bier oder Wein.

4. Brot nach Anspruch 2 oder 3, dadurch gekennzeichnet, daß die angelagerte Hefe der verwendeten Kieselgur für den Backprozeß noch aktiv ist.

5. Brot nach Anspruch 2 oder 3, dadurch gekennzeichnet, daß die angelagerte Organik der verwendeten Kieselgur durch thermische oder chemische Maßnahmen vor seiner Herstellung inaktiviert wurde, aber teilweise oder noch ganz vorhanden ist.

6. Brot nach Anspruch 2 oder 3, dadurch gekennzeichnet, daß die angelagerte Organik der verwendeten Kieselgur durch thermische oder chemische Maßnahmen vor seiner Herstellung eleminiert wurde.

7. Tabletten, gekennzeichnet durch einen Gehalt von mindestens 1 Gewichtsprozent Kieselgur aus der Filtration von flüssigen Lebensmitteln.

8. Hefetabletten, gekennzeichnet durch einen Gehalt von mindestens 1 Gewichtsprozent Kieselgur aus der Filtration von Bier oder Wein.

9. Tabletten nach Anspruch 7 oder 8, dadurch gekennzeichnet, daß das gesamte Herstellungsverfahren in einem Milieu abläuft, das ein Degenerieren der angelagerten Organik, beispielsweise der Hefe, verhindert.

10. Tabletten nach Anspruch 7 oder 8, dadurch gekennzeichnet, daß die Hefe und die andere angelagerte Organik der Kieselgur, z. B. die Hefe, durch thermische oder chemische Maßnahmen bei der Herstellung der Tabletten oder zuvor inaktiviert wurde, aber teileise oder noch ganz vorhanden ist.

11. Tabletten nach Anspruch 7 oder 8, dadurch gekennzeichnet, daß die angelagerte Organik der Kieselgur, z. B. die Hefe, durch thermische oder chemische Maßnahmen bei der Herstellung der Tabletten oder zuvor eleminiert wurde und dieser Kieselgur anschließend Hefe wieder zugemischt wird.

12. Beifuttermittel, gekennzeichnet durch Kieselgur aus der Filtration von flüssigen Lebensmitteln als Hauptmischungsbestandteil, wobei es noch andere gesundheitsfördernde Substanzen enthalten kann.

13. Beifuttermittel, gekennzeichnet durch Kieselgur aus der Filtration von Bier, Wein oder Saft als Hauptmischungsbestandteil, wobei es noch andere gesundheitsfördernde Substanzen enthalten kann.

14. Beifuttermittel nach Anspruch 12 oder 13, dadurch gekennzeichnet, daß die Organik der Kieselgur, z. B. die Hefe, noch aktiv ist.

15. Beifuttermittel nach Anspruch 12 oder 13, dadurch gekennzeichnet, daß die angelagerte Organik der Kieselgur, z. B. die Hefe, durch thermische oder chemische Maßnahmen bei seiner Herstellung oder zuvor inaktiviert wurde, aber teilweise oder noch ganz vorhanden ist.

16. Beifuttermittel nach Anspruch 12 oder 13, dadurch gekennzeichnet, daß die angelagerte Organik der Kieselgur, z. B. die Hefe, durch thermische oder chemische Maßnahmen bei seiner Herstellung oder zuvor eleminiert wurde.

17. Beifuttermittel nach Anspruch 16, gekennzeichnet durch getrocknete Bierhefe und andere gesundheitsfördernde Mischungsbestandteile.

18. Beifuttermittel nach Anspruch 12 bis 17, dadurch gekennzeichnet, daß die Kieselgur und eventuell andere gesundheitsfordernden Inhaltsstoffe, granuliert wurden.

19. Handwaschpaste, gekennzeichnet durch einen Anteil von bis zu 100 Gewichtsprozent Kieselgur aus der Filtration von flüssigen Lebensmitteln, besonders die des Bieres, bei der die angelagerte Organik denaturiert, oder durch thermische oder chemische Maßnahmen bei der Herstellung der Paste oder zuvor eleminiert, wurde.

20. Peeling-Creme, gekennzeichnet durch ein Gehalt von bis zu 95 Gewichtsprozent Kieselgur aus der Filtration von flüssigen Lebensmitteln, besonders die des Bieres, bei der die angelagerte Organik denaturiert, oder durch thermische oder chemische Maßnahmen bei der Herstellung der Creme oder zuvor eleminiert, wurde.

21. Gesichtspuder, gekennzeichnet durch einen Mischungsbestandteil von bis zu 100 Gewichtsprozent Kieselgur aus der Filtration von flüssigen Lebensmitteln, besonders die des Bieres, bei der die angelagerte Organik denaturiert, oder durch thermische oder chemische Maßnahmen bei seiner Herstellung oder zuvor eleminiert, wurde.

22. Trockenshampoo, gekennzeichnet durch einen Anteil von bis zu 100 Gewichtsprozent Kieselgur aus der Filtration von flüssigen Lebensmitteln, besonders die des Bieres, bei der die angelagerte Organik denaturiert, oder durch thermische oder chemische Maßnahmen bei seiner Herstellung oder zuvor eleminiert, wurde.

23. Schnabelwetzstein für Vögel mit einem Anteil von bis zu 90 Volumenprozent an Kieselgur aus der Filtration von flüssigen Lebensmitteln.

24. Schnabelwetzstein für Vögel mit einem Anteil von bis zu 90 Volumenprozent an Kieselgur aus der Filtration von Bier, Wein oder Saft.

25. Schnabelwetzstein für Vögel nach Anspruch 23 oder 24, dadurch gekennzeichnet, daß die Organik der Kieselgur, z. B. die Hefe, noch aktiv ist.

26. Schnabelwetzstein für Vögel nach Anspruch 23 oder 24, dadurch gekennzeichnet, daß die angelagerte Organik der Kieselgur, z. B. die Hefe, durch thermische oder chemische Maßnahmen bei seiner Herstellung oder zuvor inaktiviert wurde, aber teilweise oder noch ganz vorhanden ist.

27. Schnabelwetzstein für Vögel nach Anspruch 23 oder 24, dadurch gekennzeichnet, daß die angelagerte Organik der Kieselgur, z. B. die Hefe, durch thermische oder chemische Maßnahmen bei seiner Herstellung oder zuvor eleminiert wurde.

28. Schnabelwetzstein für Vögel nach Anspruch 27, dadurch gekennzeichnet, daß Bierhefe bzw. andere gesundheitsfördernde Substanzen, wie Vitamine oder jod, als Mischungsbestandteil zugegeben werden.

29. Tierstreu, als Beispiel Katzenstreu, gekennzeichnet durch einen Anteil von bis zu 100% Kieselgur aus der Filtration von flüssigen Lebensmitteln.

30. Tierstreu, als Beispiel Katzenstreu, dadurch gekennzeichnet, daß es bis zu 100% Kieselgur aus der Bierfiltration enthält.

31. Tierstreu nach Anspruch 29 oder 30, dadurch gekennzeichnet, daß die Kieselgur zu seiner Herstellung granuliert wird.

32. Tierstreu nach Anspruch 29 bis 31, dadurch gekennzeichnet, daß die angelagerte Organik der Kieselgur` z. B. die Hefe, durch thermische oder chemische Maßnahmen bei seiner Herstellung oder zuvor inaktiviert wurde, aber teilweise oder noch ganz vorhanden ist.

33. Tierstreu nach Anspruch 29 bis 31, dadurch gekennzeichnet, daß die angelagerte Organik der Kieselgur, z. B. die Hefe, durch thermische oder chemische Maßnahmen bei seiner Herstellung oder zuvor eleminiert wurde.

34. Tierstreu nach Anspruch 29 bis 33, bestehend Kieselgur und einem Bindemittel.

35. Tierstreu nach Anspruch 29 bis 34, gekennzeichnet durch Methylzellulose oder Wasserglas als Bindemittel zur Granulierung.

36. Tierstreu, als Beispiel Katzenstreu nach Anspruch 29 bis 35, dadurch gekennzeichnet, daß eine Substanz hinzugegeben wird, die den pH-Wert auf 5,8 bis 6,2 absenkt.

37. Tierstreu, als Beispiel Katzenstreu nach Anspruch 29 bis 36, dadurch gekennzeichnet, daß eine Substanz hinzugegeben wird, beispielsweise ein Tensid, die das Dispergiervermögen des Streus erhöht.
